# EUROPEAN PATENT SPECIFICATION

(11) **EP 3 378 462 B1**
(45) Date of publication and mention of the grant of the patent: **28.06.2023**
(21) Application number: 16863723.9
(22) Date of filing: 11.11.2016
(51) Int. Cl.: A61H 23/02, A61M 16/00, A61N 1/36

(54) **PORTABLE VEST FOR THE APPLICATION OF PULMONARY THERAPY AND ASSOCIATED METHOD**
TRAGBARE WESTE FÜR DIE ANWENDUNG VON LUNGENTHERAPIE UND ZUGEHÖRIGES VERFAHREN
GILET PORTABLE POUR L'APPLICATION DE THÉRAPIE PULMONAIRE ET PROCÉDÉ ASSOCIÉ

(30) Priority: 13.11.2015 ES 201531642
(43) Date of publication of application: 26.09.2018
(73) Proprietor: Servicio Andaluz de Salud, 41071 Sevilla (ES)
(72) Inventor: TEJERO GARCÍA, Sergio, 41071 Sevilla (ES); GIRÁLDEZ SÁNCHEZ, Miguel, Ángel, 41071 Sevilla (ES); CEJUDO RAMOS, Pilar, 41071 Sevilla (ES); QUINTANA GALLEGO, Esther, 41071 Sevilla (ES); DÍAZ GUTIÉRREZ, Fernando, 41071 Sevilla (ES); GÓMEZ SÁNCHEZ, José, Ramón, 08005 Barcelona (ES); PAJUELO LISSÉN, Eugenio, 08005 Barcelona (ES); RUBIO CARRASCO, Francisco, José, 08005 Barcelona (ES); DONCEL CAMPOS, Francisco, 41950 Castilleja de la Cuesta - Sevilla (ES); BLASCO VADILLO, Silvia, 41950 Castilleja de la Cuesta - Sevilla (ES); PINO MEJÍAS, José, Luis, 41008 Sevilla (ES); ADANERO GONZÁLEZ, Pablo, 41008 Sevilla (ES)
(74) Representative: ABG Intellectual Property Law, S.L.
(86) International application number: PCT/ES2016/070803
(87) International publication number: WO 2017/081352

(56) References cited:
- WO-A1-93/17650
- DE-A1-102005 047 219
- DE-U1-202015 001 313
- US-A1- 2002 111 571
- US-A1- 2008 000 477
- US-A1- 2010 076 518
- US-B1- 7 207 953
- DATABASE WPI Week 201155, Derwent Publications Ltd., London, GB; AN 2011-K17391, XP055380886 & WO 2011 094883 A1 (SMP SWISS MEDICAL PRODUCTS GMBH ET AL.) 11 August 2011
- DATABASE WPI Week 201341, Derwent Publications Ltd., London, GB; AN 2013-H70559, XP055380890 & CN 202 724 227 U (HU YONGSHAN ET AL.) 13 February 2013

## Description

### Object of the Invention

The present invention is comprised in the field of medicine, and more particularly in the field of devices for the treatment of chronic pulmonary diseases.

A first object of the present invention is a novel portable vest for the application of pulmonary therapy to a patient.

### Background of the Invention

Chronic pulmonary diseases are within the group of the four most relevant causes of chronic or non-communicable diseases, and according to the World Health Organization (WHO), they are responsible for 60% of deaths worldwide and for 86% in Europe. Chronic obstructive pulmonary disease (CPOD) which affects over 64 million people worldwide and over 2.18 million people in Spain and constitutes the fourth cause of death today is the most common pathology within this area. Furthermore, there are other pathologies such as cystic fibrosis, bronchiectasis, or neuromuscular diseases which likewise limit the quality of life and survival, have a serious prognosis, and furthermore entail a significant economic impact for the national health system.

One of the treatments available today for the treatment of diseases of this type consists of the application of pulmonary therapy. The purpose of pulmonary therapy is essentially to mobilize and expel the secretions retained in the bronchial tree by expanding the pulmonary tissue and promoting the efficient use of all the respiratory muscles. Some pulmonary therapy techniques such as postural drainage, percussion, chest vibration, or deep breathing and coughing exercises, for example, can be carried out by physical therapists. Alternatively, pulmonary therapy can be applied using vest-type devices designed to apply vibration to the patient's chest. This vibration causes the mobilization of secretions along the bronchial tree and enables the expulsion thereof with the help of coughing fits.

Most of the vests that are known work as a result of a series of leak-tight compartments located in the patient's chest and operated as a result of an external compressor connected to the vest through a conduit. The compressor causes the compartments to alternately inflate and deflate at a high frequency, thereby producing a mechanical vibration effect on the patient's chest mobilizing pulmonary secretions and helping to expectorate them. Patent document US6916298 describes an example of vests of this type. Pneumatic vests present the main drawback that the compressor is too heavy and bulky to be transported by the patient. For that reason, pneumatic vests usually are permanently located in a clinic and the patient has to go there every so often to receive treatment.

To solve this problem, vests in which the pneumatic compartments are replaced with electromechanical elements that apply vibration have recently emerged. These electromechanical elements are vibratory devices, such as eccentric motors, for example, which are distributed throughout the vest along the patient's chest and powered by batteries. Unlike pneumatic vests, these new electromechanical vests are lightweight and portable, which allows the patient to move around while using them, or even potentially to receive treatment at home. An example of vests of this type is described in patent document WO01/60309.

However, both pneumatic and electromechanical vests existing today present the drawback that the application of vibration treatment is continuous. This means that once they are activated, the vibration on the patient's chest is applied during both the inspiratory phase and the expiratory phase. The authors of the present invention have observed that this is counterproductive for treatment because if vibrations take place during the inspiratory phase, the pulmonary secretions that are released may tend to be located in more distal tracts, which would aggravate clinical features.

Document DE102005047219A1 discloses a tapping device for a patient's thorax, the tapping device comprising a carrier attached to the thorax, and separately controllable tapping bodies, where actions of said tapping bodies are controllable by measuring parameter and diagnostic results of patients.

In terms of features of claim 1 DE102005047219A1 discloses a portable vest for the application of pulmonary therapy, comprising a plurality of electrically activated individual vibratory elements to be arranged on the patient's chest, further comprises: a breathing detection sensor configured for identifying the expiratory phase; an actuation subsystem configured for controlling the plurality of electrically activated individual vibratory elements, and processing means configured for controlling the actuation subsystem such that the individual vibratory elements are only activated during said expiratory phase.

Document US2010/076518A1 discloses systems and methods for relieving dyspnea. In particular, a method in accordance with a particular embodiment includes receiving an input signal from a patient sensor, the input signal corresponding to an indication of the patient's breathing.

### Description of the Invention

The vest of the present invention solves the aforementioned problems as a result of it being configured particularly to work only during the patient's expiratory phase. This assures that the secretions released by the vibration tend to move along the pulmonary tree towards the upper respiratory tracts, eventually being expelled through coughing fits. Furthermore, it is a vest provided with electrically activated individual vibratory elements, so the drawbacks that pneumatic vests have due to the need to use a compressor are prevented. In this context, the term "*electrically activated*" refers to the fact that they simply require a power supply to be activated.

The invention provides a portable vest according to claim 1.

The breathing detection sensor can be configured in any way provided that it allows obtaining enough information about the patient's respiratory cycle so as to carry out the present invention to practice. This information may include the patient's respiratory rate and the times of maximum and minimum thoracic volume corresponding to the boundaries between the inspiratory and expiratory phases. For example, the breathing detection element can be in the form of a chest strap configured for detecting elongation and contraction during the patient's respiratory cycle. Electromagnetic chest straps for breathing detection of this type are currently known, such as those models manufactured by the company SleepSense, for example.

In a particularly preferred embodiment of the invention, the breathing detection sensor is configured for identifying an initial fraction of the expiratory phase, such that the individual vibratory elements are only activated during said initial fraction of the expiratory phase. Therefore during the initial fraction of the expiratory phase in which the vibratory elements are activated, the vibration applied to the patient causes the release of secretions along the patient's pulmonary tree. Once said initial fraction of the expiratory phase has ended, the vibratory elements are switched off so that during the remaining fraction of the expiratory phase, the released secretions move with greater ease towards the upper respiratory tracts. Preferably, the initial fraction of the expiratory phase is about the first two thirds of the expiratory phase.

The individual vibratory elements are located in the vest in positions corresponding to the patient's chest when the patient is wearing the vest. In this context, the term "*chest*" refers to the entire surface of the patient's rib cage, including both the patient's front and back areas. More specifically, the individual vibratory elements are located on the intercostal muscles, pectoral muscles, trapezius muscles, rhomboid muscles, levator scapulae muscles, and generally all the muscles of the torso surrounding the entire volume of the rib cage.

The individual vibratory elements can be configured in principle in any way provided that they can be activated electrically instead of pneumatically, which allows preventing the drawbacks relating to the use of a compressor. The electrodes for electrostimulation apply on the patient's skin electric pulses imitating the electric potential coming from the central nervous system, such that they cause nearby muscles to contract. The location of electrodes for electrostimulation on the patient's chest causes the muscles surrounding the patient's rib cage to contract, causing a vibratory effect similar to that exerted by the use of vibrators such as eccentric motors and the like. Furthermore, the use of electrodes for electrostimulation has the additional advantage of improving the muscular-skeletal troubles that are typical of these pulmonary pathologies. It must be observed that in order to achieve a vibratory effect when electrodes for electrostimulation are used, it is necessary to activate them in an intermittent manner.

The individual vibratory elements are divided into simultaneously activated groups distributed according to the lower-upper and/or back-front directions. In other words, the individual vibratory elements are grouped together according to their position. For example, three respectively lower, middle, and upper groups consisting of three straps around the chest can be configured. Similarly, four respectively back left, back right, front left, and front right groups essentially consisting of vertically rectangular portions can also be configured. Each of these groups of vibratory elements is connected such that all the vibratory elements of the group are activated simultaneously. This configuration allows selectively activating the groups of vibratory elements in a given order during each cycle to optimize the release of secretions from the pulmonary tree.

The simultaneously activated groups are selectively actuated such that the vibration is applied in the lower-upper and/or back-front direction as the expiratory phase progresses. Applying vibration in the lower-upper direction as the expiratory phase progresses means that the vibration starts to be activated at the beginning of the vibration cycle, i.e., at the beginning of the expiratory phase, in the simultaneously activated lower groups and progressively moves towards the simultaneously activated upper groups while the vibration lasts. This has the effect of *"pushing"* the released secretions upwards towards the upper respiratory tracts. Likewise, the application of the vibration in the back-front direction means that the vibration starts to be activated at the beginning of the vibration cycle in the simultaneously activated back groups and progressively moves towards the simultaneously activated front groups while the vibration lasts. This has the effect of *"pushing"* the released secretions from the back part of the respiratory tree towards the front part, thereby helping to bring them closer to the upper respiratory tracts.

In another preferred embodiment of the invention, the individual vibratory elements can be selectively actuated such that the intensity of the vibration tapers off as the expiratory phase progresses. In other words, the applied vibration has a maximum intensity at the beginning of the vibration cycle, i.e., at the beginning of the expiratory phase, and said intensity tapers off until adopting a minimum intensity at the end of the vibration cycle. The intensity can progressively taper off in a smooth manner, for example linearly, or it can taper off in a step-wise manner. The achieved effect is that the secretions are detached especially in the first expiratory phase in order to be driven in a centrifugal manner due to expiratory flow and not in a centripetal manner due to inspiratory flow.

In another preferred embodiment of the invention, the vest further comprises at least one sensor for sensing at least one variable representative of the patient's physical state so as to allow stopping the individual vibratory elements should the value of said variable exceed a certain threshold. An independent sensor can be used for each of the monitored variables, or multi-variable sensors capable of measuring more than one variable can be used. For example, the sensor can be a heart rate sensor, a blood oxygen saturation level sensor, or a combination thereof. The use of these sensors allows monitoring the values of variables representative of the patient's physical state in order to determine if the patient is in a physical state suitable for receiving treatment. In the event of detecting that the patient's state worsens, for example a dangerous rise in heart rate or an abnormal drop in the blood oxygen saturation level, suspension of the treatment can be done automatically (i.e., the individual vibratory elements are commanded to stop). This feature is an additional safety measure that is very suitable for the use of the vest of the invention in the patient's home without medical supervision.

In yet another preferred embodiment, the vest of the invention further comprises a thoracic volume restricting element. The thoracic volume restricting element can be configured in any way provided that it imposes an upper limit on the patient's inspiration volume. For example, a non-extendable chest strap that can be adjusted to different measurements can be used. The possibility of restricting the thoracic volume allows selecting the site of action of the therapy: more proximal in the case of a smaller inspiration volume limit, and more distal in the case of a greater inspiration volume limit. In other words, the thoracic volume restricting element allows selectively directing the action of the vest on the upper or lower respiratory tracts. There is currently no therapeutic vest capable of carrying out a similar function.

In another preferred embodiment of the invention, the vest further comprises a communication means configured for transmitting information about the operation to an external monitoring device. This allows medical personnel to periodically download the operating data from the vest in order to verify the treatment the patient has received.

In another preferred embodiment of the invention, the vest is water-resistant. This means that it is configured to withstand being immersed in water, such that the patient can use it in a pool while swimming, for example. When the patient receives therapy at the same time he or she practices physical exercise, small respiratory tracts that are not affected when at rest are cleared. However, a significant percentage of patients requiring therapies of this type are elderly patients, so virtually the only risk-free physical exercise they can practice is swimming or water exercises in general. So since the vest is water-resistant, vibration therapy can be applied to these patients while they swim, and it is therefore possible for them to work on the respiratory cycle, the muscles of the torso, and the postural drainage of secretions at the same time.

Another aspect of the present disclosure relates to a method for operating a vest like the one described in preceding paragraphs for the application of pulmonary therapy, where the method comprises the step of activating the individual vibratory elements only during the expiratory phase, more preferably only during a first fraction of the expiratory phase, and even more preferably only during about the first two thirds of the expiratory phase.

In another preferred embodiment, the method comprises the step of selectively activating the individual vibratory elements such that the vibration is applied in distal-proximal and/or back-front direction.

In another preferred embodiment, the method comprises causing the intensity of the vibration to taper off during each expiration.

In yet another preferred embodiment, the method further comprises the step of selectively restricting the patient's thoracic volume.

In yet another preferred embodiment, the method further comprises the steps of detecting at least one physical variable representative of the patient's physical state and detaining the operation of the individual vibratory elements should said physical variable exceed certain thresholds.

In another preferred embodiment of the method, the step of activating the individual vibratory elements comprises applying electrostimulation pulses in an intermittent manner.

### Brief Description of the Drawings

Figures 1 and 2 respectively show a front perspective view and a rear perspective view of an exemplary vest according to the present invention.
Figures 3 and 4 respectively show a front perspective view and a rear perspective view of a vest where the simultaneously activated groups can be seen in greater detail.
Figures 5a and 5b show two steps of an exemplary operating cycle of the vest where the simultaneously activated groups are actuated in the lower-upper direction.
Figures 6a and 6b show two steps of an exemplary operating cycle of the vest where the simultaneously activated groups are actuated in the back-front direction.
Figure 7 shows a front perspective view of a vest where two breathing detection elements can be seen in greater detail.
Figure 8 shows a front perspective view of a vest where a thoracic volume restricting element can be seen in greater detail.
Figure 9 shows a diagram of the different functional blocks forming the vest of the invention.

### Preferred Embodiments of the Invention

An example of a portable vest (1) according to the present invention is described below in reference to the attached drawings. When the positions of the different elements constituting the vest (1) are described herein making reference to parts of the patient's body, it is understood that it is the position said elements take in relation to the patient's body when he or she is suitably wearing the vest (1). For example, if it is stated that the vest (1) has a given element in the right pectoral area, this means that said element is arranged in a position of the vest (1) such that when the patient is wearing the vest (1), it is located on the patient's right pectoral area.

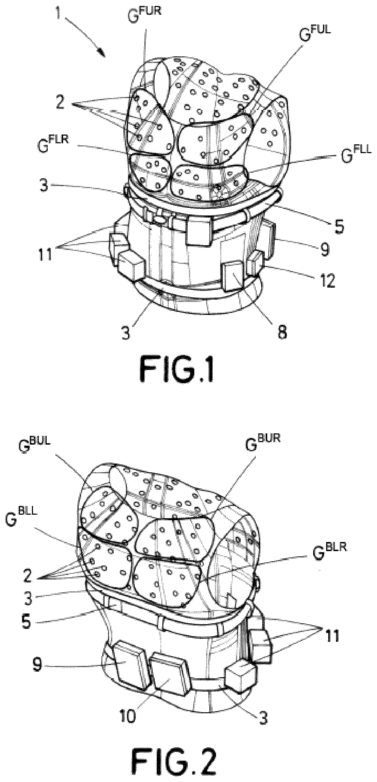

Figures 1 and 2 respectively show a front view and rear view of an exemplary vest (1) according to the present invention where the main components forming it can be seen. The vest (1) is essentially formed by a type of chest plate made of a multilayer textile fabric on which a plurality of individual vibratory elements (2) is arranged. The chest plate has no sleeves and covers the upper part of the patient's torso including the chest, and in this example, the abdominal and lumbar portion as well. The chest plate part located in the abdominal and lumbar area, where vibration is not applied, is used in this example for fixing auxiliary elements that will be described below.

As mentioned above, the individual vibratory elements (2) can be mechanical elements, such as eccentric motors or the like, or they can be electrodes for electrostimulation. In any case, the individual vibratory elements (2) are distributed throughout the patient's entire chest in both the front and back areas.

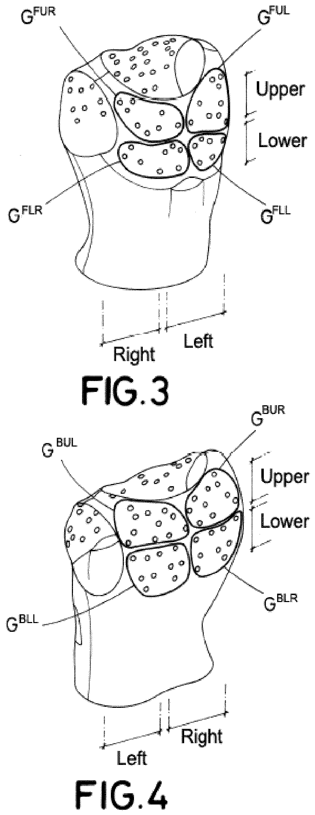

In this example, the individual vibratory elements (2) are furthermore grouped together in several simultaneously activated groups (G^{ijk}) according to the lower-upper and/or back-front directions. According to the notation used herein, in each group (G^{ijk}) the indices (i, j, k) can respectively take the values (front/back, upper/lower, left/right). As depicted in Figures 3 and 4, four simultaneously activated groups (G^{ijk}) are arranged in the front area of the vest (1), specifically a front upper right group (G^{FUR}), a front upper left group (G^{FUL}), a front lower right group (G^{FLR}), and a front lower left group (G^{FLL}), and four other simultaneously activated groups (G^{ijk}) are arranged in the back area of the vest (1), specifically a back upper right group (G^{BUR}), a back upper left group (G^{BUL}), a back lower right group (G^{BLR}), and a back lower left group (G^{BLL}).

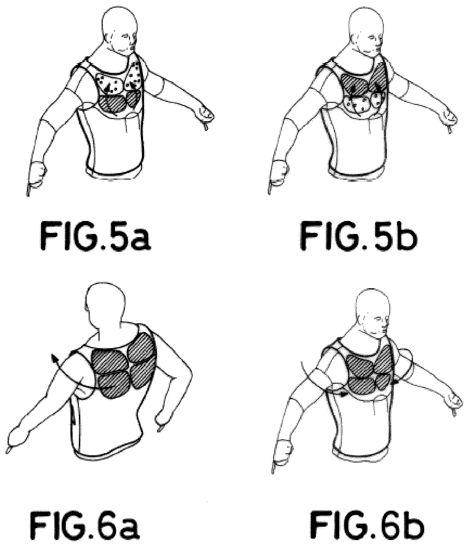

This configuration allows simultaneously activating at will the lower portion of the vest (1) if the lower groups (G^{FLR}, G^{FLL}, G^{BLR}, G^{BLL}) are activated, as depicted in Figure 5a, or the upper portion of the vest (1) if the upper groups (G^{FUR}, G^{FUL}, G^{BUR}, G^{BUL}) are activated, as depicted in Figure 5b. If this is done sequentially in each vibration cycle, first activating the lower groups (G^{FLR}, G^{FLL}, G^{BLR}, G^{BLL}) and then the upper groups (G^{FUR}, G^{FUL}, G^{BUR}, G^{BUL}), a bottom-to-top *"pushing"* effect is created on the released secretions, pushing them towards the patient's upper airways to make the expulsion thereof easier.

Alternatively, the back portion of the vest (1) can be simultaneously activated at will if the back groups (G^{BUR}, G^{BUL}, G^{BLR}, G^{BLL}) are activated, as depicted in Figure 6a, or the front portion of the vest (1) can be simultaneously activated at will if the front groups (G^{FUR}, G^{FUL}, G^{FLR}, G^{FLL}) are activated, as depicted in Figure 6b. If this is done sequentially in each cycle, first activating the back groups (G^{BUR}, G^{BUL}, G^{BLR}, G^{BLL}) and then the front groups (G^{FUR}, G^{FUL}, G^{FLR}, G^{FLL}), a back-to-front *"pushing"* effect is created on the released secretions, bringing them closer to the patient's upper airways.

It must be observed that it would be possible to configure the vest (1) with any number and configuration of simultaneously activated groups (G^{ijk}) arranged according to the lower-upper and/or back-front directions. The larger the number of simultaneously activated groups (G^{ijk}), the more precise the control of the *"pushing"* effect that is obtained will be. Furthermore, it would be possible to configure the activation of the simultaneously activated groups (G^{ijk}) such that a back-front *"pushing"* effect and at the same time a lower-upper *"pushing"* effect are exerted.

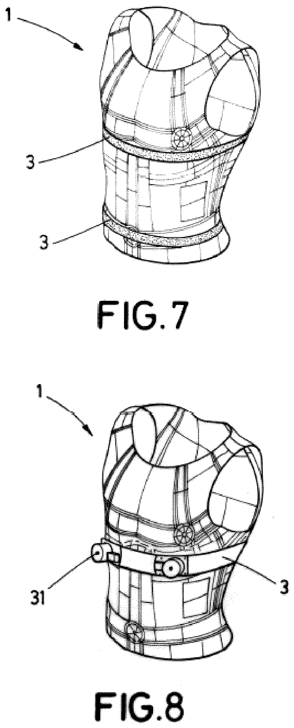

The vest (1) shown in Figures 1 and 2 furthermore has two breathing detection sensors (3) taking the form of electromagnetic straps arranged around the patient's torso respectively in the pectoral area and in the abdominal area. This can be seen in greater detail in Figure 7, where all the components of the vest (1) except for the two breathing detection sensors (3) have been eliminated. The use of two detection sensors (3) allows obtaining with greater precision information about the patient's respiratory cycle, which is known to comprise a first pectoral phase and a second abdominal phase. Specifically, the two breathing detection sensors (3) in the form of a strap allow counting the number of breaths as well as recording the tidal volume or amount of air circulating through the respiratory tree in a cycle.

As a result, the patient's respiratory rate and the moments during which the transition from inspiration to expiration and from expiration to inspiration takes place are known after a calibration period. As mentioned hereinabove, the authors of the present invention have discovered that the results obtained by this treatment are better if the vibration of the vest (1) is activated only during the expiratory phase. The information obtained by the breathing detection sensors (3) allows activating the vibration of the individual vibratory elements (2) at the time during which the transition from inspiration to expiration takes place, and deactivating them at the very latest when the transition from expiration to inspiration takes place. As discussed hereinabove, the secretions released during the expiratory phase are thereby prevented from tending to more deeply penetrate the patient's pulmonary tree during the inspiratory phase.

Furthermore, the authors of the present invention have discovered that the results improve even more if the vibration is detained before the patient's expiratory phase ends. In other words, the individual vibratory elements (2) are activated during an initial fraction of the expiratory phase, and then deactivated to remain in the inactive state during the remaining final fraction of said expiratory phase and the entire inspiratory phase. For example, the individual vibratory elements (2) can be kept activated during the first two thirds of the expiratory phase and remain deactivated during the last third. Secretions are thereby released first as a result of the vibration applied by the vest (1) during the initial fraction of the expiratory phase, and the expulsion of the secretions during the remaining final fraction of the expiratory phase is then made easier.

The authors of the present invention have also observed that the results can be even further improved if the intensity of the vibration in each vibration cycle is progressively decreased. In other words, the individual vibratory elements (2) are activated at the beginning of expiration with a first vibration intensity, and as the expiratory phase progresses the vibration intensity decreases. As explained above, this has the effect of releasing secretions by taking advantage of the higher expiratory flow to move secretions in a centrifugal manner for the purpose of making bronchial hygiene therapy more efficient.

Figures 1 and 2 also depict a thoracic volume restricting element (5). As can be seen with greater clarity in Figure 8, where the rest of the elements of the vest (1) of the invention have been eliminated, the thoracic volume restricting element (5) of this example is configured as a non-extendable strap having an adjustable length arranged in the vest (1) at the height of the patient's chest. Wheels (31) arranged in the front portion the element (5) allow adjusting its length for the purpose of limiting the patient's thoracic volume according to different levels depending on the respiratory tracts that are to be worked on at all times. When the thoracic volume is restricted, the opening or closing of the more distal tracts means that the therapy takes place in a different location of the bronchial-alveolar tree.

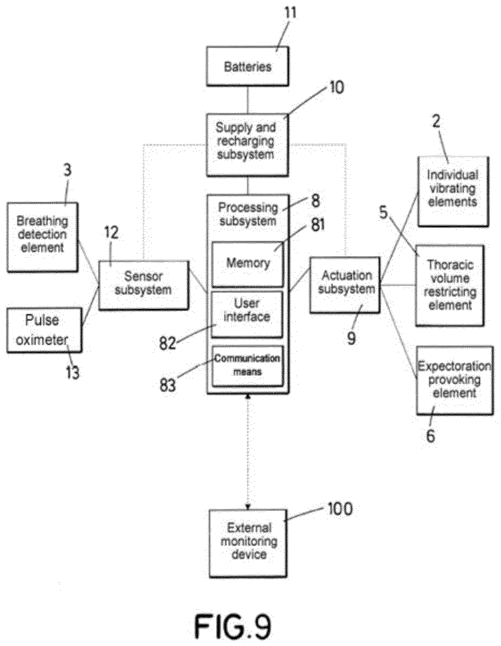

Finally, Figure 9 shows a diagram showing a possible configuration of the different functional blocks making up the vest (1) of the invention. Each functional block, or subsystem, is formed by means of electronics suitable for carrying out the functions commissioned in each case. For example, these electronics can be microcontrollers, microprocessors, DSPs, ASICs, FPGAs, or any other device suited to that effect. In this example, each of these functional blocks is fixed to the chest plate in a given position in the abdominal area which is free of vibratory elements (2), as discussed above. It must be observed that this specific configuration of the functional blocks is merely a non-limiting implementing example of the invention, and that it would be possible to structure these blocks differently.

The function of the actuation subsystem (9) is to control operation of the different actuators of the system, which in this example are the vibratory elements (2), the thoracic volume restricting element (5), and the expectoration provoking element (6). The actuation subsystem (9) primarily determines when the different actuators (2, 5, 6) are activated and deactivated, as well as the operating mode during which they are activated and deactivated.

The function of the supply and recharging subsystem (10) is to control the operation of the batteries (11). It is primarily in charge of managing the voltage provided by the batteries (11) and controlling the process of charging the batteries from an external supply.

The function of the sensor subsystem (12) is to control the operation of the different sensors used, which in this example are the breathing detection element (3) and the pulse oximeter (13). In this example, the breathing detection element (3) provides both information about the respiratory rate and the moments of transition between inspiration and expiration, as well as the patient's heart rate. The sensor subsystem (12) therefore primarily receives signals from the sensors (3, 13), processes them, and sends them in a format that can be used by the rest of the system.

Finally, the function of the processing means (8) is to manage the overall operation of the vest (1), which includes control of the aforementioned subsystems (9, 11, 12), as well as having other functions. The processing means (8) comprise a memory (81) which allows storing the data regarding the therapy applied to the patient so that it can subsequently be consulted by the doctor. Furthermore, the processing means (8) a user interface (82) that can be provided with a display and a keyboard or any other equivalent data input element, such that the patient or the doctor can select the therapy to be applied by the vest (1) and display the relevant data. Additionally, the processing means (8) also comprise a communication means (83), preferably wireless communication means (83), which allow exchanging data with an external monitoring device (100). This allows the doctor to consult the data stored in the memory (81) when the patient who has been treated at home with the vest (1) goes to the doctor's office. For example, the external monitoring device (100) can be a computer, a tablet, or a smartphone. The scope of the invention is defined by the appended claims.

## Claims

1. A portable vest (1) for the application of pulmonary therapy, comprising a plurality of electrically activated individual vibratory elements (2) to be arranged on the patient's chest, wherein the vibratory elements (2) are electrodes for electrostimulation configured for being activated in an intermittent manner such that a vibratory effect is induced on the patient's chest;
**characterized in that** it further comprises:
a breathing detection sensor (3) configured for identifying the expiratory phase;
an actuation subsystem (9) configured for controlling the plurality of electrically activated individual vibratory elements (2), and
processing means (8) configured for controlling the actuation subsystem (9) such that the individual vibratory elements (2) are only activated during said expiratory phase, and
**in that** said individual vibratory elements (2) are divided into simultaneously activated groups (G^{ijk}) distributed according to the lower-upper and/or back-front directions, wherein the simultaneously activated groups (G^{ijk}) are selectively actuated such that the vibration is applied at the beginning of the expiratory phase in the simultaneously activated lower and/or back groups and progressively moves towards the simultaneously activated upper and/or front groups while the expiratory phase lasts.

2. The vest (1) according to claim 1, wherein the breathing detection sensor (3) is configured for identifying an initial fraction of the expiratory phase, such that the individual vibratory elements (2) are only activated during said initial fraction of the expiratory phase.

3. The vest (1) according to claim 2, wherein the initial fraction of the expiratory phase identified by the breathing detection sensor (3) is about the first two thirds of said expiratory phase.

4. The vest (1) according to any of the preceding claims, wherein the individual vibratory elements (2) are selectively actuated such that the intensity of the vibration has a maximum intensity at the beginning of the expiratory phase, and said intensity tapers off until adopting a minimum intensity at the end of the expiratory phase.

5. The vest (1) according to any of the preceding claims, further comprising at least one sensor (4) of at least one variable representative of the patient's physical state so as to allow stopping the individual vibratory elements (2) should the value of said variable exceed a certain threshold.

6. The vest (1) according to claim 5, wherein the sensor (4) is chosen from: a heart rate sensor, a blood oxygen saturation level sensor, and a combination thereof.

7. The vest (1) according to any of the preceding claims, further comprising a thoracic volume restricting element (5).

8. The vest (1) according to any of the preceding claims, further comprising a communication means (83) configured for transmitting information about the operation to an external monitoring device (100).

## Patentansprüche

1. Tragbare Weste (1) für die Anwendung einer Lungentherapie, die eine Vielzahl von elektrisch aktivierten einzelnen Vibrationselementen (2) aufweist, die auf der Brust des Patienten anzuordnen sind, wobei die Vibrationselemente (2) Elektroden zur Elektrostimulation sind, die dazu eingerichtet sind, in einer intermittierenden Weise aktiviert werden, so dass ein Vibrationseffekt auf der Brust des Patienten induziert wird;
**dadurch gekennzeichnet, dass** sie ferner aufweist:
einen Atmungsdetektionssensor (3), der zum Identifizieren der Exspirationsphase eingerichtet ist;
ein Betätigungssubsystem (9), das zum Steuern der Vielzahl von elektrisch aktivierten einzelnen Vibrationselementen (2) eingerichtet ist, und
Verarbeitungsmittel (8), die zum Steuern des Betätigungssubsystems (9) eingerichtet sind, so dass die einzelnen Vibrationselemente (2) nur während der Exspirationsphase aktiviert werden, und
dadurch, dass die einzelnen Vibrationselemente (2) in gleichzeitig aktivierte Gruppen (G^{ijk} ) unterteilt sind, die entsprechend den unten-oben und/oder hinten-vorne Richtungen verteilt sind, wobei die gleichzeitig aktivierten Gruppen (G^{ijk} ) selektiv so betätigt werden, dass die Vibration zu Beginn der Exspirationsphase in den gleichzeitig aktivierten unteren und/oder hinteren Gruppen aufgebracht wird und sich während der Dauer der Exspirationsphase progressiv zu den gleichzeitig aktivierten oberen und/oder vorderen Gruppen bewegt.

2. Weste (1) nach Anspruch. 1, wobei der Atmungsdetektionssensor (3) zum Identifizieren eines Anfangsanteils der Exspirationsphase eingerichtet ist, so dass die einzelnen Vibrationselemente (2) nur während dieses Anfangsanteils der Exspirationsphase aktiviert werden.

3. Weste (1) nach Anspruch 2, wobei der Anfangsanteil der Exspirationsphase, der von dem Atmungsdetektionssensor (3) identifiziert wird, etwa die ersten zwei Drittel der Exspirationsphase beträgt.

4. Weste (1) nach einem der vorhergehenden Ansprüche, wobei die einzelnen Vibrationselemente (2) selektiv so betätigt werden, dass die Intensität der Vibration zu Beginn der Exspirationsphase eine maximale Intensität hat und diese Intensität abnimmt, bis sie am Ende der Exspirationsphase eine minimale Intensität annimmt.

5. Weste (1) nach einem der vorhergehenden Ansprüche, ferner aufweisend mindestens einen Sensor (4) mindestens einer Variable, die für den körperlichen Zustand des Patienten repräsentativ ist, um ein Anhalten der einzelnen Vibrationselemente (2) zu ermöglichen, wenn der Wert dieser Variable einen bestimmten Schwellenwert überschreitet.

6. Weste (1) nach Anspruch 5, wobei der Sensor (4) ausgewählt ist aus: einem Herzfrequenzsensor, einem Blutsauerstoffsättigungssensor und einer Kombination deren.

7. Weste (1) nach einem der vorhergehenden Ansprüche, ferner aufweisend ein Brustraumvolumen-Begrenzungselement (5).

8. Weste (1) nach einem der vorhergehenden Ansprüche, ferner aufweisend ein Kommunikationsmittel (83), das zum Übertragen von Informationen über den Betrieb an eine externe Überwachungsvorrichtung (100) eingerichtet ist.

## Revendications

1. Un gilet portable (1) pour l'application d'une thérapie pulmonaire, comprenant une pluralité d'éléments vibratoires individuels (2) activés électriquement destinés à être agencés sur la poitrine d'un patient, les éléments vibratoires (2) étant des électrodes d'électrostimulation configurées pour être activées de manière intermittente de sorte qu'un effet vibratoire soit induit sur la poitrine du patient ;
**caractérisé en ce qu'**il comprend en outre :
un capteur de détection de respiration (3) configuré pour identifier la phase expiratoire ;
un sous-système d'actionnement (9) configuré pour commander la pluralité d'éléments vibratoires individuels (2) activés électriquement, et
des moyens de traitement (8) configurés pour commander le sous-système d'actionnement (9) de sorte que les éléments vibratoires individuels (2) ne soient activés que pendant ladite phase expiratoire, et
**en ce que** lesdits éléments vibratoires individuels (2) sont divisés en groupes activés simultanément (G^{ijk}) répartis selon les directions inférieur-supérieur et/ou arrière-avant, les groupes activés simultanément (G^{ijk}) étant actionnés sélectivement de sorte que la vibration soit appliquée au début de la phase expiratoire dans les groupes inférieurs et/ou arrières activés simultanément et qu'elle se déplace progressivement vers les groupes supérieurs et/ou avant activés simultanément pendant la durée de la phase expiratoire.

2. Le gilet (1) selon la revendication 1, dans lequel le capteur de détection de respiration (3) est configuré pour identifier une fraction initiale de la phase expiratoire, de sorte que les éléments vibratoires individuels (2) ne sont activés que pendant ladite fraction initiale de la phase expiratoire.

3. Le gilet (1) selon la revendication 2, dans lequel la fraction initiale de la phase expiratoire identifiée par le capteur de détection de respiration (3) est d'environ les deux premiers tiers de ladite phase expiratoire.

4. Le gilet (1) selon l'une quelconque des revendications précédentes, dans lequel les éléments vibratoires individuels (2) sont actionnés sélectivement de sorte que l'intensité de la vibration ait une intensité maximale au début de la phase expiratoire, et que ladite intensité décroisse jusqu'à adopter une intensité minimale à la fin de la phase expiratoire.

5. Le gilet (1) selon l'une quelconque des revendications précédentes, comprenant en outre au moins un capteur (4) d'au moins une variable représentative de l'état physique du patient de manière à permettre d'arrêter les éléments vibratoires individuels (2) si la valeur de ladite variable dépasse un certain seuil.

6. Le gilet (1) selon la revendication 5, dans lequel le capteur (4) est choisi parmi : un capteur de fréquence cardiaque, un capteur de niveau de saturation en oxygène du sang, et une combinaison de ceux-ci.

7. Le gilet (1) selon l'une quelconque des revendications précédentes, comprenant en outre un élément de restriction de volume thoracique (5).

8. Le gilet (1) selon l'une quelconque des revendications précédentes, comprenant en outre un moyen de communication (83) configuré pour transmettre des informations sur le fonctionnement à un dispositif de surveillance externe (100).
